# EUROPÄISCHE PATENTSCHRIFT

(11) **EP 0 393 545 B1**
(45) Veröffentlichungstag und Bekanntmachung des Hinweises auf die Patenterteilung: **07.07.1999**
(21) Anmeldenummer: 90107148.0
(22) Anmeldetag: 14.04.1990
(51) Int. Cl.: C08G 18/10, C08G 18/66, C08G 18/76, A61F 2/00, A61M 5/00

(54) **Glasklare heissdampfsterilisierbare kompakte Polyurethan-Vergussmassen, Verfahren zu ihrer Herstellung und ihre Verwendung, insbesondere für medizinisch technische Artikel**
Crystalclear, vapour sterilisable compact polyurethane casting compounds, process for their preparation and their use, especially for medical-technical articles
Masses à couler de polyuréthane compacts, clairs et stérilisables à la vapeur chaude, procédé pour leur préparation et leur utilisation, en particulier pour des articles techniques médicinaux

(30) Priorität: 17.04.1989 DE 3912531
(43) Veröffentlichungstag der Anmeldung: 24.10.1990
(73) Patentinhaber: BASF Aktiengesellschaft, 67063 Ludwigshafen (DE)
(72) Erfinder: Horn, Peter, Dr., D-6900 Heidelberg (DE); Heckmann, Walter, Dr., D6940 Weinheim (DE); Ramsteiner, Falko, Dr., D-6700 Ludwigshafen (DE); Gerold, Friedrich, D-8013 Haar (DE)

(56) Entgegenhaltungen:
- EP-A- 0 111 121
- DE-A- 2 749 491
- FR-A- 2 380 309
- US-A- 4 224 164
- US-A- 4 814 103

## Beschreibung

Erfindungsgegenstand sind glasklare, heißdampfsterilisierbare, im wesentlichen kompakte Polyurethan- - im folgenden auch abgekürzt PU genannt - Vergußmassen, die hergestellt werden durch Umsetzung von
A) modifizierten Diphenylmethan-diisocyanaten - im folgenden auch abgekürzt MDI genannt -, die ihrerseits erhalten werden durch Umsetzung
   A1) einer MDI-Mischung, die bezogen auf 100 Gew.-Teile, besteht aus
      A11) 60 bis 90 Gew.-Teilen 4,4'-MDI,
      A12) 40 bis 8 Gew.-Teilen 2,4'-MDI und
      A13) 0 bis 5 Gew.-Teilen 2,2'-MDI mit
   A2) mindestens einem mit Glycerin- und/oder Trimethylolpropan gestarteten Polyoxypropylen-polyol mit einem Molekulargewicht von 350 bis 800 im Verhältnis von NCO-:OH-Gruppen von 2,5:1 bis 15:1,
      mit
B) einer Mischung aus Verbindungen mit mindestens zwei reaktiven Wasserstoffatomen, die besteht aus
   B1) mindestens einer Polyhydroxylverbindung mit einem Molekulargewicht von 1000 bis 8500 und einer Funktionalität von 2 bis 4, ausgewählt aus der Gruppe der Polyetherole, Polyesterole, hydroxylgruppenhaltigen Polyacetale und hydroxylgruppenhaltigen Polycarbonate,
   B2) mindestens einer Dihydroxyverbindung, ausgewählt aus der Gruppe der Alkandiole mit 6 bis 19 C-Atomen, der Ester- und Ethergruppen gebundenen enthaltenden Glykole mit Molekulargewichten bis 378 und der Oxyalkylenglykole mit 4 bis 8 C-Atomen und
   B3) mindestens einem hydroxylgruppenhaltigen Vernetzer mit einer Hydroxylzahl von 230 bis 1900 und einer Funktionalität von 3 bis 8,
      wobei das Molverhältnis von B1:B2:B3 = 1:0,01 bis 48:0,01 bis 32 beträgt.
   C) Katalysatoren.

PU-Gießsysteme sind bekannt und werden z.B. zusammenfassend beschrieben im Kunststoff-Handbuch "Polyurethane", Band 7, 2. Auflage, 1983, Seiten 392ff, herausgegeben von Dr. G. Oertel, im Carl Hanser Verlag, München, Wien.

Die Verwendung von PU-Vergußmassen zur Herstellung von Formteilen für medizinisch technische Geräte, insbesondere als Einbettwerkstoff für Hohlfasern in Dialysatoren, ist ebenfalls nicht neu und wird aufgrund der einfachen Handhabung von PU-Vergußmassen sowie deren geringer Schrumpfung während des Aushärtungsprozesses als vorteilhaft empfohlen. Bekannt sind z.B. folgende PU-Formulierungen speziell zur Einbettung von Hohlfasern.

In der US-A-3 962 094 werden katalysatorfreie Vergußmassen aus Rizinusöl-4,4'-MDI-, -Toluylen-diisocyanat- oder -Phenylen-diisocyanat-Prepolymeren mit endständigen NCO-Gruppen und einem Vernetzungsmittel, das enthält Rizinusöl und/oder einen Ester aus einem mindestens vierwertigen Alkohol und einer Hydroxy- oder Epoxygruppen aufweisenden aliphatischen Carbonsäure mit mindestens 12 Kohlenstoffatomen, beschrieben.

Nach Angaben der DE-A-2 749 491 (US-A-4 170 559) bestehen die katalysatorfreien Vergußmassen aus einem Prepolymeren, hergestellt aus Rizinusöl und Polyoxypropylen-glykol sowie 4,4'-MDI, und einem Vernetzungsmittel auf der Grundlage eines Esters aus einem mehrwertigen Alkohol mit 2 oder 3 Hydroxylgruppen und einer aliphatischen Carbonsäure mit mindestens 12 C-Atomen und einer oder mehreren Hydroxyl- und/oder Epoxygruppen. Als geeignete Polyisocyanate zur Herstellung der Prepolymeren werden ferner genannt: 2,4- und 2,6-Toluylen-diisocyanat oder Phenylen-diisocyanat. Als Vernetzungsmittel kommen außerdem Monoester und/oder Diester von Ethylenglykol und Ricinolsäure, Trimethylolpropan oder -ethan in Betracht.

Physiologisch unbedenkliche PU-Formstoffe, insbesondere zum Einbetten von Hohlfasern in Dialysatoren, werden nach Angaben der DD-A-251 565 hergestellt durch Umsetzung hochreaktiver, niedrigviskoser und lagerstabiler Mischprepolymerer, bestehend aus festen, hochreaktiven aromatischen Diisocyanaten und weniger reaktiven, flüssigen Diisocyanaten im Gewichtsverhältnis von 1:5 bis 5:1 und Polyolen, mit Polyolen aus der Gruppe Rizinusöl und/oder dessen Umesterungsprodukte, hochreiner Polyester und Polyoxytetramethylen-glykol. PU-Vergupmassen aus einem Isocyanatendgruppen aufweisenden PU-Prepolymeren und einer N,N,N',N'-Tetrakis(2-hydroxypropyl)-ethylendiamin enthaltenden Polyolmischung sind Gegenstand des US-Patents Nr. 4 224 164. Nach Angaben der US-A-4 742 112 finden zur Herstellung von PU-Vergußmassen für elektrische Geräte als Polyolkomponente Mischungen aus 10 bis 60 Gew.% eines Ricinolsäureesters und 90 bis 40 Gew.% eines C₂- bis C₆-Kohlenwasserstoffpolymeren mit mindestens einer Hydroxylgruppe Verwendung. Zweikomponenten PU-Formulierungen, die im gehärteten Zustand nicht cytotoxisch sind und sich als Vergußmassen für Trennvorrichtungen eignen, bestehen nach Angaben der DE-A-3 048 529 (US-A-4 332 927) aus mindestens einem NCO-terminiertem Prepolymeren, mindestens einem Polyol und einer katalytisch wirksamen Mengen einer dicarboxylierten Dialkylzinnverbindung. Mit Zinn-Schwefel-Verbindungen katalysierte PU-Vergußmassen zur Einbettung von Cellulosehohlfasern in Dialysatoren werden beschrieben in der DD-A-155 777.

Die vorgenannten PU-Vergußmassen können zu medizinisch-technischen Geräten oder Formteilen hierfür verarbeitet und vor ihrem Gebrauch mit Ethylenoxid oder/und mit γ-Strahlen sterilisiert werden. Nachteilig an dieser Art der Sterilisation ist jedoch, dap verbleibende Ethylenoxidspuren bei Patienten teilweise Allergien auslösen können und die γ-Strahlen nicht identifizierbare Spaltprodukte bilden können, so daß beim Patienten ein gewisses Gesundheitsrisiko, verursacht durch die Dialyse, nicht vollständig ausgeschlossen werden kann. Die nach dem Stand der Technik bekannten Vergußmassen sind jedoch nicht ausreichend temperatur- und chemikalienresistent, so daß diese keiner Heißdampfsterilisation bei einer Temperatur von 121°C über einen Zeitraum von 20 Minuten unterworfen werden können.

Die Aufgabe der vorliegenden Erfindung bestand darin, glasklare, im wesentlichen kompakte, heißdampfsterilisierbare PU-Vergußmassen für medizinisch-technische Artikel zu entwickeln, die sich insbesondere zum Einbetten von Hohlfasern, vorzugsweise solchen auf Basis von Polysulfonen, Polycarbonaten oder Cellulose, in Dialysezellen eignen und einen festen Verbund mit dem Gehäuse, das in der Regel aus einem Bisphenol-A-polycarbonat besteht, gewährleisten. Die PU-Vergußmasse muß rasch durchhärten, darf im ausgehärteten Zustand keine Wechselwirkung zu den eingelegten Hohlfasern zeigen und darf nicht toxisch sein.

Diese Aufgabe konnte überraschenderweise gelöst werden durch die Verwendung eines bei Raumtemperatur flüssigen, modifizierten Diphenylmethandiisocyanat-Isomerengemisches zur Herstellung der PU-Vergußmassen.

Gegenstand der Erfindung sind somit glasklare, im wesentlichen kompakte, heißdampfsterilisierbare PU-Vergußmassen, die hergestellt werden durch Umsetzung von
A) modifizierten Diphenylmethan-diisocyanaten mit
B) einer Mischung aus Verbindungen mit mindestens zwei reaktiven Wasserstoffatomen, die besteht aus
   B1) mindestens einer Polyhydroxylverbindung mit einem Molekulargewicht von 1000 bis 8500 und einer Funktionalität von 2 bis 4, ausgewählt aus der Gruppe der Polyetherole, Polyesterole, hydroxylgruppenhaltigen Polyacetale und hydroxylgruppenhaltigen Polycarbonate,
   B2) mindestens einer Dihydroxyverbindung, ausgewählt aus der Gruppe der Alkandiole mit 6 bis 19 C-Atomen, der Ester- und Ethergruppen gebundenen enthaltenden Glykole mit Molekulargewichten bis 378 und der Oxyalkylenglykole mit 4 bis 8 C-Atomen und
   B3) mindestens einem hydroxylgruppenhaltigen Vernetzer mit einer Hydroxylzahl von 230 bis 1900 und einer Funktionalität von 3 bis 8,
      wobei das Molverhältnis von B1:B2:B3 = 1:0,01 bis 48:0,01 bis 32 beträgt.
C) Katalysatoren
und dadurch gekennzeichnet sind, daß die modifizierten Diphenylmethan-diisocyanate (A) bei 23°C flüssig sind und hergestellt werden durch Umsetzung
A1) einer Diphenylmethan-diisocyanat-Isomerenmischung, die bezogen auf 100 Gew.-Teile, bestehend aus
   A11) 60 bis 90 Gew.-Teilen, vorzugsweise 70 bis 88 Gew.-Teilen 4,4'-diphenylmethan-Diisocyanat,
   A12) 40 bis 8 Gew.-Teilen, vorzugsweise 30 bis 10 Gew.-Teilen 2,4'-Diphenylmethan-diisocyanat und
   A13) 0 bis 5 Gew.-Teilen, vorzugsweise 0 bis 3 Gew.-Teilen 2,2'-Diphenylmethan-diisocyanat
      mit
A2) mindestens einem Polyoxypropylen-polyol mit einem Molekulargewicht von 350 bis 800, vorzugsweise von 400 bis 700, erhalten unter Verwendung von Glycerin, Trimethylolpropan oder einer Mischung aus Glycerin und Trimethylolpropan als Startermoleküle,
   im Verhältnis von NCO-:OH-Gruppen von 2,5:1 bis 15:1, vorzugsweise von 5:1 bis 10:1.

Gegenstände der Erfindung sind ferner ein Verfahren zur Herstellung der glasklaren, heißdampfsterilisierbaren, im wesentlichen kompakten Polyurethan-Vergußmassen gemäß Anspruch 9 und die Verwendung der PU-Vergußmassen zum Einbetten von Hohlfasern aus vorzugsweise Polysulfonen, Polycarbonaten oder Cellulose in Dialysatoren, zur Herstellung von medizinisch-technischen Artikeln sowie zur Bindung von Biokeramikbeschichtungen auf Endoprothesen nach Anspruch 10.

Da im Stand der Technik als geeignete Polyisocyanate zur Herstellung der PU-Vergußmassen, insbesondere zum Einbetten von Hohlfasern in Dialysatoren, neben 1,5-Naphthylen-diisocyanat, Toluylen-diisocyanaten und Phenylen-diisocyanaten auch 4,4'-MDI genannt wird, die Polyisocyanate zweckmäßigerweise in Form von Prepolymeren zur Reaktion gebracht werden und hierbei keine heipdampfsterilisierbaren Polyurethane erhalten werden, war überraschend und nicht vorhersehbar, daß die ausgewählte, spezielle MDI-Isomerenmischung, modifiziert mit den speziellen Polyoxypropylentriolen in bestimmten Mengenverhältnissen den hieraus hergestellten, ausgehärteten PU-Vergußmassen eine erhöhte Temperaturbeständigkeit und verbesserte Hydrolysebeständigkeit verleihen, so daß die medizinisch-technischen Artikel problemlos heißdampfsterilisiert werden können.

Vorteilhaft ist ferner, daß die erfindungsgemäßen PU-Vergußmassen beim Aushärten die Maximaltemperatur von 127°C, gemessen im Mittelpunkt eines konisch sich öffnenden 300 ml Bechers aus Hartpapier (der Firma Uniplast, 7417 Dillingen, BRD) mit einem Bodendurchmesser von ca. 53 mm und einem öffnungsdurchmesser von ca. 75 mm, in den 100 g Reaktionsmischung eingefüllt werden, nicht erreichen, da bei Temperaturen über 127°C die Hohlfasern geschädigt werden.
A) Die erfindungsgemäß verwendbaren modifizierten MDI (A) besitzen bei 23°C zweckmäßigerweise eine Viskosität von 1000 bis 3000 m·Pa·s, vorzugsweise von 1200 bis 2000 m·Pa·s und einen NCO-Gehalt von 17 bis 26 Gew.%, vorzugsweise von 19 bis 24 Gew.%, bezogen auf das Gesamtgewicht und werden hergestellt nach an sich bekannten Verfahren durch Umsetzung der MDI-Isomerenmischung (A1) mit mindestens einem Polyoxypropylen-triol (A2) bei einer Temperatur von zweckmäßigerweise 60 bis 100°C, vorzugsweise 70 bis 90°C und einer Reaktionszeit von 0,5 bis 3 Stunden, vorzugsweise von 1 bis 2 Stunden.
   Als Polyoxypropylen-polyole (A2) kommen in Betracht: mit Glycerin gestartete Polyoxypropylen-polyole, mit Trimethylolpropan gestartete Polyoxypropylen-polyole oder Mischungen der genannten Polyoxypropylen-polyole. Gleichermaßen geeignet sind auch die Polyoxypropylen-polyole, hergestellt unter Verwendung einer Mischung aus Glycerin und Trimethylolpropan als Startermoleküle, wobei die Gewichtsverhältnisse von Glycerin zu Trimethylolpropan in breiten Grenzen variiert werden können. Vorzugsweise verwendet wird ein mit Glycerin gestartetes Polyoxypropylen-triol mit einem Molekulargewicht von ungefähr 420.
B) Als Verbindungen mit mindestens zwei reaktiven Wasserstoffatomen (B) werden Mischungen verwendet, die bestehen aus
   B1) mindestens einer Polyhydroxylverbindung mit einem Molekulargewicht von 1000 bis 8500 und einer Funktionalität von 2 bis 8,
   B2) mindestens einem niedermolekularen zweiwertigen Alkohol, Ester- und/oder Etherbrücken gebunden enthaltenden Glykol, und
   B3) mindestens einem hydroxylgruppenhaltigen Vernetzer mit einer Hydroxylzahl von 230 bis 1900 und einer Funktionalität von 3 bis 8.
   B1) Als Polyhydroxylverbindungen (B1) mit einem Molekulargewicht von 1000 bis 8500, vorzugsweise von 1500 bis 5600 und insbesondere von 1800 bis 4000 und einer Funktionalität von 2 bis 8, vorzugsweise 2 bis 4 und insbesondere 2 und/oder 3 eignen sich vorzugsweise Polyesterole und insbesondere Polyetherole. In Betracht kommen jedoch auch andere hydroxylgruppenhaltige Polymere mit Ether- oder Estergruppen als Brückenglieder, beispielsweise Polyacetale, wie Polyoxymethylene und vor allem wasserunlösliche Formale, z.B. Polybutandiolformal und Polyhexandiolformal, und Polycarbonate, insbesondere solche aus Diphenylcarbonat und Hexandiol-1,6, hergestellt durch Umesterung. Die genannten Polyhydroxylverbindungen können als Einzelkomponenten oder in Form von Mischungen zur Anwendung kommen.
      Geeignete Polyesterole können beispielsweise aus Dicarbonsäuren mit 2 bis 12, vorzugsweise 4 bis 6 Kohlenstoffatomen und mehrwertigen Alkoholen hergestellt werden. Als Dicarbonsäuren kommen beispielsweise in Betracht: aliphatische Dicarbonsäuren, wie Bernsteinsäure, Glutarsäure, Adipinsäure, Korksäure, Azelainsäure und Sebacinsäure und aromatische Dicarbonsäuren, wie Phthalsäure, Isophthalsäure und Terephthalsäure. Die Dicarbonsäuren können einzeln oder als Gemische, z.B. in Form einer Bernstein-, Glutar- und Adipinsäuremischung, verwendet werden. Zur Herstellung der Polyesterole kann es gegebenenfalls vorteilhaft sein, anstelle der Dicarbonsäuren die entsprechenden Dicarbonsäurederivate, wie Dicarbonsäuremono- oder -diester mit 1 bis 4 Kohlenstoffatomen im Alkoholrest, Dicarbonsäureanhydride oder Dicarbonsäuredichloride zu verwenden. Beispiele für mehrwertige Alkohole sind Glykole mit 2 bis 10, vorzugsweise 2 bis 6 Kohlenstoffatomen, wie Ethylenglykol, Diethylenglykol, Butandiol-1,4, Pentandiol-1,5, Hexandiol-1,6, Decandiol-1,10, 2,2-Dimethylpropandiol-1,3, Propandiol-1,3 und Dipropylenglykol. Je nach den gewünschten Eigenschaften können die mehrwertigen Alkohole allein oder gegebenenfalls in Mischungen untereinander verwendet werden.
      Geeignet sind ferner Ester der Kohlensäure mit den genannten Diolen, insbesondere solchen mit 4 bis 6 Kohlenstoffatomen, wie Butandiol-1,4 und/oder Hexandiol-1,6, Kondensationsprodukte von ω-Hydroxycarbonsäuren, beispielsweise ω-Hydroxycapronsäure und vorzugsweise Polymerisationsprodukte von Lactonen, beispielsweise gegebenenfalls substituierten ω-Caprolactonen.
      Als Polyesterole vorzugsweise verwendet werden Ethandiol-polyadipate, l,4-Butandiol-polyadipate, Ethandiol-1,4-butandiol-polyadipate, 1,6-Hexandiol-neopentylglykol-polyadipate, -polyadipate, 1,6-Hexandiol-1,4-butandiolpolyadipate und Polycaprolactone.
      Die Polyesterole besitzen Molekulargewichte von 1500 bis 5600, vorzugsweise von 1800 bis 3500.
      Die insbesondere bevorzugt verwendeten Polyetherole können nach bekannten Verfahren, beispielsweise durch anionische Polymerisation mit Alkalihydroxiden, wie Natrium- oder Kaliumhydroxid oder Alkalialkoholaten, wie Natriummethylat, Natrium- oder Kaliumethylat oder Kaliumisopropylat als Katalysatoren und unter Zusatz mindestens eines Startermoleküls das 2 bis 8, vorzugsweise 2 bis 4 reaktive Wasserstoffatome gebunden enthält, oder durch kationische Polymerisation mit Lewis-Säuren, wie Antimonpentachlorid, Borfluorid-Etherat u.a. oder Bleicherde als Katalysatoren aus einem oder mehreren Alkylenoxiden mit 2 bis 4 Kohlenstoffatomen im Alkylenrest hergestellt werden.
      Geeignete Alkylenoxide sind beispielsweise Tetrahydrofuran, 1,3-Propylenoxid, 1,2- bzw. 2,3-Butylenoxid und bevorzugt Ethylenoxid und 1,2-Propylenoxid. Die Alkylenoxide können einzeln, alternierend nacheinander oder als Mischungen verwendet werden. Als Startermoleküle kommen beispielsweise in Betracht: Wasser, organische Dicarbonsäuren, wie Bernsteinsäure, Adipinsäure und/oder Glutarsäure, Alkanolamine, wie z.B. Ethanolamin, N-Alkylalkanolamine, N-Alkyl-dialkanolamine, wie z.B. N-Methyl- und N-Ethyl-diethanolamin und vorzugsweise zwei- oder dreiwertige, gegebenenfalls Etherbrücken gebunden enthaltende Alkohole, wie z.B. Ethandiol, Propandiol-1,2 und -1,3, Butandiol-1,4, Diethylenglykol, Pentandiol-1,5, Hexandiol-1,6, Dipropylenglykol, 2-Methyl-pentandiol-1,5 und 2-Ethyl-butandiol-1,4, Glycerin, Trimethylolpropan, Pentaerythrit, Sorbit und Saccharose. Die Startermoleküle können einzeln oder als Gemische eingesetzt werden.
      Vorzugsweise verwendet werden Polyetherole aus 1,2-Propylenoxid und Ethylenoxid, in denen mehr als 50 %, vorzugsweise 60 bis 80 % der OH-Gruppen primäre Hydroxylgruppen sind und bei denen zumindest ein Teil des Ethylenoxids als endständiger Block angeordnet ist. Derartige Polyetherole können erhalten werden, indem man z.B. an das Startermolekül zunächst das 1,2-Propylenoxid und daran anschließend das Ethylenoxid polymerisiert oder zunächst das gesamte 1,2-Propylenoxid im Gemisch mit einem Teil des Ethylenoxids copolymerisiert und den Rest des Ethylenoxids anschließend anpolymerisiert oder schrittweise zunächst einen Teil des Ethylenoxids, dann das gesamte 1,2-Propylenoxid und dann den Rest des Ethylenoxids an das Startermolekül anpolymerisiert.
      Insbesondere geeignet sind ferner Polyoxytetramethylen-glykole, vorteilhafterweise solche mit Molekulargewichten von 1000 bis 3000.
      Die geeigneten Polyetherole besitzen Molekulargewichte von 1000 bis 8500, vorzugsweise 1500 bis 5600 und insbesondere 1800 bis 4000. Sie können sowohl einzeln als auch in Form von Mischungen untereinander zur Anwendung kommen.
      Als hydroxylgruppenhaltige Polyacetale kommen z.B. die aus Glykolen, wie Diethylenglykol, Triethylenglykol, 4,4'-Dihydroxyethoxy-diphenyldimethylmethan, Hexandiol und Formaldehyd herstellbaren Verbindungen in Frage. Auch durch Polymerisation cyclischer Acetale lassen sich geeignete Polyacetale herstellen.
      Als Hydroxylgruppen aufweisende Polycarbonate kommen solche der an sich bekannten Art in Betracht, die beispielsweise durch Umsetzung von Diolen, wie Propandiol-1,3, Butandiol-1,4 und/oder Hexandiol-1,6, Diethylenglykol, Triethylenglykol oder Tetraethylenglykol mit Diarylcarbonaten, z.B. Diphenylcarbonat, oder Phosgen hergestellt werden können.
   B2) Als niedermolekulare zweiwertige Alkohole, Ester- oder Ethergruppen als Brückenglieder gebunden enthaltende Glykole kommen z.B. in Betracht: Alkandiole mit 2 bis 10 C-Atomen, vorzugsweise 2 bis 6 C-Atomen, wie z.B. Ethandiol, Propandiol-1,2 oder -1,3, 2,2-Dimethyl-propandiol, Butandiol-1,4, -1,3 oder -2,3, Pentandiol-1,5 oder -2,5, Hexandiol-1,6, 2,2,5-Trimethyl- oder 2,2,5,5-Tetramethyl-hexandiol-1,6; Cycloalkandiole und Alkylcycloalkandiole mit 6 bis 19 C-Atomen, vorzugsweise 6 bis 15 C-Atomen, wie z.B. 1,4-Dihydroxy-cyclohexan, 1-Hydroxymethyl-4-hydroxycyclohexan, 1,4-Bis-(hydroxymethyl)-cyclohexan, 4,4'-Dihydroxy-dicyclohexyl-methan oder -propan-2,2, Esterbrücken gebunden enthaltende Glykole, wie z.B. 3-Hydroxy-2,2-dimethylpropionsäure-2-hydroxyethylester, Terephthalsäure-bis-ethylenglykol oder -butandiol-1,4 und Etherbrücken gebunden enthaltende Glykole mit Molekulargewichten bis 378, wie z.B. Hydroxyalkylenether des Hydrochinons, beispielsweise 1,4-Di-(β-hydroxyethyl)-hydrochinon, Oxyalkylen-glykol mit 4 bis 8 C-Atomen, wie z.B. Diethylen-, Dipropylen- oder Dibutylen-glykol sowie die entsprechenden höhermolekularen Oligomeren davon, wie z.B. Dioxyethylen-, Trioxyethylen-, Dioxypropylen-, Trioxypropylen-, Dioxybutylen-, Trioxybutylen- oder Tetraoxybutylen-glykol. Die Dihydroxyverbindungen aus der Gruppe der Alkan-, Cycloalkan-, Alkylcycloalkyl-diole und der entsprechenden Ester- oder Etherbrücken gebunden enthaltenden Glykole können einzeln oder als Mischungen eingesetzt werden.
   B3) Als hydroxylgruppenhaltige Vernetzer mit einer Hydroxylzahl von 230 bis 1900 und einer Funktionalität von 3 bis 8 finden vorzugsweise niedermolekulare 3- bis 8-wertige, vorzugsweise 3- bis 4-wertige Alkohole, Trialkanolamine, mit Alkylen-diaminen mit zweckmäßigerweise 2 bis 6 C-Atomen gestartete Polyoxyalkylen-polyole mit Hydroxylzahlen von 350 bis 950 oder mit niedermolekularen 3- bis 8-wertigen Alkoholen gestartete Polyoxyalkylen-polyole mit Hydroxylzahlen von 230 bis 1500 Verwendung. Beispielhaft genannt seien als 3- und höherwertige Alkohole: Glycerin, Trimethylolpropan, Pentaerythrit, 2,2,6,6-Tetrahydroxymethyl-4-oxaheptandiol-1,7 (Di-pentaerythrit), Tripentaerythrit, 3,3,7,7-Tetrahydroxymethyl-5-oxanon (Di-Trimethylolpropan) und Sorbit, Trialkanolamine wie Triethanolamin und als mit Alkylen-diaminen gestarteten Polyoxyalkylen-polyole die Umsetzungsprodukte von 1,2-Propylenoxid, Ethylenoxid oder von Mischungen aus Ethylenoxid und 1,2-Propylenoxid mit aromatischen oder vorzugsweise aliphatischen Diaminen, wie z.B. Ethylendiamin, 1,3-Propylendiamin, 1,3- bzw. 1,4-Butylendiamin, 1,2-, 1,3-, 1,4-, 1,5- und l,6-Hexamethylendiamin, Phenylendiamine, 2,4- und 2,6-Toluylendiamin und 4,4'-, 2,4'- und 2,2'-diamino-diphenylmethan oder mit Diethylentriamin, Triethylentetramin. Als Polyether-polyole, hergestellt aus Verbindungen der erwähnten Gruppe sind besonders interessant N,N,N',N'-Tetrakis-(2-hydroxyethyl)-ethylendiamin, N, N,N',N'-Tetrakis-(2-hydroxypropyl)-ethylendiamin, N,N,N',N",N"-Pentakis-(2-hydroxypropyl)-diethylentriamin. zur Herstellung der Polyoxyalkylen-polyole mit Hydroxylzahlen von 230 bis 1500 finden die vorgenannten 3- bis 8-wertigen Alkohole als Startermoleküle und Ethylenoxid und/oder 1,2-Propylenoxid Verwendung.
      Die Polyhydroxylverbindungen B1 bis B3 werden in solchen Mengen verwendet, daß die Mischung B besteht aus 1,0 Molen B1, 0,01 bis 48 Molen, vorzugsweise 2 bis 20 Molen B2 und 0,01 bis 32 Molen, vorzugsweise 1,3 bis 7 Molen B3.
      Gegebenenfalls kann es vorteilhaft sein, insbesondere wenn PU-Vergußmassen mit hervorragender Oberflächenqualität erforderlich sind, zusätzlich zu den obengenannten Polyhydroxylverbindungen (B1) bis (B3) als weitere Aufbaukomponente (D) zusätzlich zu verwenden: Glycerinmonooleat, Glycerindioleat oder ein Alkylepoxystearat mit einem verzweigtkettigen Alkylrest mit mindestens 8 C-Atomen, vorzugsweise 10 bis 20 C-Atomen oder Mischungen aus mindestens zwei der genannten Verbindungen. Sofern die Glycerinmono- und/oder -dioleate und/oder Alkylepoxystearate Anwendung finden, werden diese zweckmäßigerweise in einer Menge von 0,1 bis 5 Gew.%, vorzugsweise von 1 bis 4 Gew.%, bezogen auf das Gesamtgewicht von (A) und (B), eingesetzt.

Die Herstellung der PU-Vergußmassen kann in Gegenwart oder Abwesenheit von Katalysatoren durchgeführt werden. Als geeignete Katalysatoren haben sich Dialkylcarboxylate wie z.B. Dibutylzinndiacetat, Dibutylzinndilaurat sowie dicarboxylierte Dialkylzinnverbindungen, wie sie in der DE-A-3 048 529 beschrieben werden, bewährt. Sofern Katalysatoren eingesetzt werden, werden diese üblicherweise in einer Menge von 0,001 bis 0,1 Gew.-Teilen, vorzugsweise 0,005 bis 0,015 Gew.-Teilen pro 100 Gew.-Teile der Aufbaukomponente (B) verwendet.

Zur Herstellung der PU-Vergußmassen werden die modifizierten MDI (A) und Verbindungen mit mindestens zwei reaktiven Wasserstoffatomen (B) sowie gegebenenfalls die Aufbaukomponente (D) in Gegenwart oder Abwesenheit der Katalysatoren (C) in solchen Mengen zur Umsetzung gebracht, daß das Äquivalenz-Verhältnis von NCO-Gruppen der modifizierten MDI (A) zur Summe der reaktiven Wasserstoffatome der Komponente (B) und gegebenenfalls (D) 1:0,9 bis 1,3, vorzugsweise 1:0,95 bis 1,15 und insbesondere 1:0,98 bis 1,05 beträgt. Hierzu werden die im wesentlichen vollständig entgasten Ausgangskomponenten bei Temperaturen von zweckmäßigerweise 18 bis 70°C, vorzugsweise von 22 bis 60°C intensiv gemischt, die Reaktionsmischung in ein geeignetes Formwerkzeug eingebracht und über einen Zeitraum von 0,3 bis 4 Stunden, vorzugsweise von 1 bis 3 Stunden aushärten gelassen.

Wie bereits dargelegt wurde, finden die glasklaren, im wesentlichen kompakten, heißdampfsterilisierbaren PU-Vergußmassen insbesondere Verwendung, zum Einbetten von Hohlfasern, vorzugsweise von Polysulfon-, Polycarbonat- oder Cellulosehohlfasern in Dialysatoren, wobei die Dialysegeräte, insbesondere die Hülse für das Dialysefilter, zweckmäßigerweise aus einem Polycarbonat auf Basis von Bisphenol A besteht.

Die erfindungsgemäßen PU-Vergußmassen eignen sich ferner zur Herstellung von medizinisch-technischen Artikeln und zur Bindung von Biokeramikbeschichtungen auf Endoprothesen.

Die PU-Vergußmassen sind nicht toxisch, glasklar, zeigen keine Wechselwirkung mit den Hohlfasern, besitzen eine starke Haftung zum Polycarbonat und sind ohne. Zerstörung der eingebetten Hohlfasern gut zerschneidbar. Wesentlich für die Verwendung der Produkte ist ferner, daß die Maximaltemperatur bei der Aushärtung unter den genannten Bedingungen unter 127°C liegt und die medizinisch-technischen Artikel der Heißdampfsterilisation unterworfen werden können, ohne daß die ausgehärtete PU-Vergußmasse oder deren Haftung an das Polycarbonatgehäuse geschädigt wird.

### Beispiel 1

### Herstellung des modifizierten MDI

In einem 4 Liter Dreihalskolben wurde eine MDI-Mischung, bestehend aus

| | |
|---|---|
| 1093,56 g | 4,4' -MDI und |
| 156,23 g | 2,4' -MDI |

auf 80°C erwärmt und hierzu unter Rühren

| | |
|---|---|
| 250,21 g | eines mit Glycerin gestarteten Polyoxypropylen-polyols mit einer Hydroxylzahl von 400 |

über einen Zeitraum von 60 Minuten zutropfen gelassen. Zur Vervollständigung der Umsetzung wurde anschließend noch 60 Minuten bei 80°C gerührt. Das erhaltene modifizierte MDI besaß einen NCO-Gehalt von 22,88 Gew.% und eine Viskosität bei 25°C von 1170 m·Pa·s.

### Beispiel 2

### Herstellung der PU-Vergußmassen

### A-Komponente:

Mischung aus

| | |
|---|---|
| 85,0 | Gew.-Teilen eines mit Trimethylolpropan gestarteten Polyoxypropylen (86 Gew.%)-polyoxyethylen (14 Gew.%)-triols mit der Hydroxylzahl 28, |
| 2,0 | Gew.-Teilen eines mit Ethylendiamin gestarteten Polyoxypropylen (85 Gew.%)-polyoxyethylen (15 Gew.%)-tetrols mit der Hydroxylzahl 60, |
| 10,0 | Gew.-Teilen Butandiol-1,4, |
| 3,0 | Gew.-Teilen Glycerin und |
| 0,01 | Gew.-Teilen Dibutylzinndilaurat. |

### B-Komponente:

modifiziertes MDI, hergestellt nach den Angaben des Beispiels 1.

100 Gew.-Teile der Komponente A und 70,2 Gew.-Teile der Komponente B wurden bei 23°C intensiv gemischt, die Reaktionsmischung in ein Formwerkzeug eingegossen und aushärten gelassen.

Die Gelzeit betrug 180 Sekunden und die Maximaltemperatur 97,3°C, gemessen im Mittelpunkt eines konisch sich öffnenden 300 ml Hartpapierbechers mit einem Bodendurchmesser von 53 mm und einem öffnungsdurchmesser von 75 mm, in den 100 ml Reaktionsmischung eingefüllt wurden. Die PU-Vergußmasse war glasklar und beständig gegen Wasserdampf bei 121°C über einen Zeitraum von mehr als 20 Minuten.

Die Heißdampfsterilisation von Dialysatoren aus Polycarbonat, bestückt mit Polysulfonhohlfasern, hergestellt nach dem Schleudergußverfahren unter Verwendung der PU-Vergupmasse gemäß Beispiel 2, ergab keinerlei Beschädigung.

### Beispiel 3

### A-Komponente:

Mischung aus

| | |
|---|---|
| 84,0 | Gew.-Teilen eines mit Trimethylolpropan gestarteten Polyoxypropylen (86 Gew.%)-polyoxyethylen (14 Gew.%)-triols mit der Hydroxylzahl 28, |
| 14,0 | Gew.-Teilen Pentandiol-1,5, |
| 2,0 | Gew.-Teilen Glycerin und |
| 0,015 | Gew.-Teilen Dibutylzinndilaurat. |

### B-Komponente:

75,2 Gew.-Teile modifiziertes MDI, hergestellt nach Beispiel 1.

Zur Herstellung der PU-Vergußmasse wurde analog den Angaben von Beispiel 2 verfahren.

Die Gelzeit betrug 132 Sekunden und die Maximaltemperatur 104°C.

Das glasklare Produkt wurde durch die Heißdampfsterilisation nicht geschädigt.

### Beispiel 4

### A-Komponente:

Mischung aus

| | |
|---|---|
| 80,0 | Gew.-Teilen eines mit Trimethylolpropan gestarteten Polyoxypropylen (86 Gew.%)-polyoxyethylen (14 Gew.%)-triols mit der Hydroxylzahl 27, |
| 10,0 | Gew.-Teilen Butandiol-1,4 |
| 10,0 | Gew.-Teilen eines mit Ethylendiamin gestarteten Polyoxypropylen-tetrols mit der Hydroxylzahl 768 und |
| 0,005 | Gew.-Teilen Dibutylzinndilaurat. |

### B-Komponente:

80,6 Gew.-Teile modifiziertes MDI, hergestellt nach Beispiel 1.

Zur Herstellung der PU-Vergußmasse wurde analog den Angaben von Beispiel 2 verfahren.

Die Gelzeit betrug 171 Sekunden und die Maximaltemperatur 94°C .

Das glasklare Produkt wurde durch die Heißdampfsterilisation nicht geschädigt.

### Beispiel 5

### A-Komponente:

Mischung aus

| | |
|---|---|
| 82,0 | Gew.-Teilen eines mit Trimethylolpropan gestarteten Polyoxypropylen (86 Gew.%)-polyoxyethylen (14 Gew.%)-triols mit der Hydroxylzahl 26, |
| 10,0 | Gew.-Teilen Pentandiol-1,5, |
| 1,0 | Gew.-Teilen Glycerin, |
| 7,0 | Gew.-Teilen eines mit Trimethylolpropan gestarteten Polyoxyethylen-triols mit der Hydroxylzahl 944 und |
| 0,015 | Gew.-Teilen Dibutylzinndilaurat. |

### B-Komponente:

76,7 Gew.-Teile modifiziertes MDI, hergestellt nach Beispiel 1.

Zur Herstellung der PU-Vergußmasse wurde analog den Angaben von Beispiel 2 verfahren:

Die Gelzeit betrug 130 Sekunden und die Maximaltemperatur 102°C.

Das glasklare Produkt wurde durch die Heißdampfsterilisation nicht geschädigt.

### Beispiel 6

### A-Komponente:

Mischung aus

| | |
|---|---|
| 85,0 | Gew.-Teilen eines mit Trimethylolpropan gestarteten Polyoxypropylen (86 Gew.%)-polyoxyethylen (14 Gew.%)-triols mit der Hydroxylzahl 26, |
| 5,0 | Gew.-Teilen Butandiol-1,4 |
| 5,0 | Gew.-Teilen Glycerin, |
| 5,0 | Gew.-Teilen eines mit Ethylendiamin gestarteten Polyoxypropylen-tetrols mit der Hydroxylzahl 768 und |
| 0,01 | Gew.-Teilen Dibutylzinndilaurat. |

### B-Komponente:

77,0 Gew.-Teile modifiziertes MDI, hergestellt nach Beispiel 1.

Zur Herstellung der PU-Vergußmasse wurde analog den Angaben von Beispiel 2 verfahren.

Die Gelzeit betrug 161 Sekunden und die Maximaltemperatur 97°C .

Das glasklare Produkt wurde durch die Heißdampfsterilisation nicht geschädigt.

### Vergleichsbeispiel

Man verfuhr analog den Angaben des Beispiels 1 des US-A-4 224 164.

Man erhielt eine klare, gelbliche PU-Vergußmasse, die sich unter den Bedingungen der Heißdampfsterilisation vom Polycarbonatgehäuse ablöste.

## Patentansprüche

1. Glasklare, heißdampfsterilisierbare, im wesentlichen kompakte Polyurethan-Vergußmassen, hergestellt durch Umsetzung von
A) modifizierte Diphenylmethan-diisocyanaten mit
B) einer Mischung aus Verbindungen mit mindestens zwei reaktiven Wasserstoffatomen, die besteht aus
B1) mindestens einer Polyhydroxylverbindung mit einem Molekulargewicht von 1000 bis 8500 und einer Funktionalität von 2 bis 4, ausgewählt aus der Gruppe der Polyetherole, Polyesterole, hydroxylgruppenhaltigen Polyacetale und hydroxylgruppenhaltigen Polycarbonate,
B2) mindestens einer Dihydroxyverbindung, ausgewählt aus der Gruppe der Alkandiole mit 2 bis 10 C-Atomen, der (Alkyl)cycloalkandiole mit 6 bis 19 C-Atomen, der Ester- oder Etherbrücken gebunden enthaltenden Glykole mit Molekulargewichten bis 378 und der Oxyalkylenglykole mit 4 bis 8 C-Atomen und
B3) mindestens einem hydroxylgruppenhaltigen Vernetzer mit einer Hydroxylzahl von 230 bis 1900 und einer Funktionalität von 3 bis 8, wobei das Molverhältnis von B1:B2:B3 = 1:0,01 bis 48:0,01 bis 32 beträgt,
im Äquivalenzverhältnis NCO-Gruppen von (A) zur Summe der reaktiven Wasserstoffatome von (B) von 1:0,09 bis 1,3
in Gegenwart oder Abwesenheit von
C) Katalysatoren
dadurch gekennzeichnet, daß die modifizierten Diphenylmethandiisocyanate (A) bei 23°C flüssig sind und hergestellt werden durch Umsetzung
A1) einer Diphenylmethan-diisocyanat-Isomerenmischung, die bezogen auf 100 Gew.-Teile, besteht aus
A11) 60 bis 90-Gew.-Teilen 4,4'Diphenylmethan-diisocyanat,
A12) 40 bis 8 Gew.-Teilen 2,4'-Diphenylmethan-diisocyanat und
A13) 0 bis 5 Gew.-Teilen 2,2'Diphenylmethan-diisocyanat mit
A2) mindestens einem Polyoxypropylenpolyol mit einem Molekulargewicht von 350 bis 800, erhalten unter Verwendung von Glycerin, Trimethylolpropan oder einer Mischung aus Glycerin und Trimethylolpropan als Startermoleküle,
im Verhältnis von NCO-:OH-Gruppen von 2,5:1 bis 15:1.

2. Verfahren zur Herstellung von glasklaren, heißdampfsterilisierbaren, im wesentlichen kompakten Polyurethan-Vergußmassen durch Umsetzung von
A) modifizierten Diphenylmethan-diisocyanaten mit
B) einer Mischung aus Verbindungen mit mindestens zwei reaktiven Wasserstoffatomen, die besteht aus
B1) mindestens einer Polyhydroxylverbindung mit einem Molekulargewicht von 1000 bis 8500 und einer Funktionalität von 2 bis 4, ausgewählt aus der Gruppe der Polyetherole, Polyesterole, hydroxylgruppenhaltigen Polyacetale und hydroxylgruppenhaltigen Polycarbonate,
B2) mindestens einer Dihydroxyverbindung, ausgewählt aus der Gruppe der Alkandiole mit 2 bis 10 C-Atomen, der (Alkyl)cycloalkandiole mit 6 bis 19 C-Atomen, der Ester- oder Etherbrücken gebunden enthaltenden Glykole mit Molekulargewichten bis 378 und der Oxyalkylenglykole mit 4 bis 8 C-Atomen und
B3) mindestens einem Vernetzer mit einer Hydroxylzahl von 230 bis 1900 aus der Gruppe der niedermolekularen 3-bis 8-wertigen Alkohole, Trialkanolamine, mit Alkylendiaminen gestarteten Polyoxyalkylen-polyole mit Hydroxylzahlen von 350 bis 950 oder mit niedermolekularen 3- bis 8-wertigen Alkoholen gestarteten Polyoxyalkylenpolyolen mit Hydroxylzahlen von 944 bis 1500
B3) mindestens einem hydroxylgruppenhaltigen Vernetzer mit einer Hydroxylzahl von 230 bis 1900 und einer Funktionalität von 3 bis 8, wobei das Molverhältnis von B1:B2:B3 = 1:0,01 bis 48:0,01 bis 32 beträgt,
im Äquivalenzverhältnis von NCO-Gruppen von (A) zur Summe der reaktiven Wasserstoffatome von (B) von 1:0,9 bis 1,3
in Gegenwart oder Abwesenheit von
C) Katalysatoren
dadurch gekennzeichnet, daß die modifizierten Diphenylmethandiisocyanate (A) bei 23°C flüssig sind und hergestellt werden durch Umsetzung
A1) einer Diphenylmethan-diisocyanat-Isomerenmischung, die bezogen auf 100 Gew.-Teile, besteht aus
A11) 60 bis 90 Gew.-Teilen 4,4'-Diphenylmethan-diisocyanat
A12) 40 bis 8 Gew.-Teilen 2,4'-Diphenylmethan-diisocyanat und
A13) 0 bis 5 Gew.-Teilen 2,2'-Diphenylmethan-diisocyanat mit
A2) mindestens einem Polyoxypropylenpolyol mit einem Molekulargewicht von 350 bis 800, erhalten unter Verwendung von Glycerin, Trimethylolpropan oder einer Mischung aus Glycerin und Trimethylolpropan als Startermoleküle,
im Verhältnis von NCO-:OH-Gruppen von 2,5:1 bis 15:1.

3. Verwendung der glasklaren, heißdampfsterilisierbaren, im wesentlichen kompakten Polyurethan-Vergußmassen nach Anspruch 1 zum Einbetten von Hohlfasern aus vorzugsweise Polysulfonen, Polycarbonaten oder Cellulose in Dialysegeräte, zur Herstellung von medizinisch-technischen Artikeln sowie zur Bindung von Biokeramikbeschichtungen auf Endoprothesen.

## Claims

1. A crystal clear, autoclaveable, essentially compact polyurethane embedding composition, prepared by reacting
A) modified diphenylmethane diisocyanates with
B) a mixture of compounds having at least two reactive hydrogen atoms, which consists of
B1) at least one polyhydroxy compound with a molecular weight of from 1000 to 8500 and a functionality of from 2 to 4, selected from the group of polyetherols, polyesterols, hydroxyl-containing polyacetals and hydroxyl-containing polycarbonates,
B2) at least one dihydroxy compound selected from the group of alkanediols having 2 to 10 C atoms, of (alkyl)cycloalkanediols having 6 to 19 C atoms, of glycols which contain bound ester or ether bridges and have molecular weights of up to 378 and of oxyalkylene glycols having 4 to 8 C atoms and
B3) at least one hydroxyl-containing crosslinker with a hydroxyl number of from 230 to 1900 and a functionality of from 3 to 8, where the B1:B2:B3 molar ratio is 1:0.01 - 48:0.01 - 32,
in the equivalence ratio of NCO groups of (A) to the total of the reactive hydrogen atoms of (B) of 1:0.09 - 1.3
in the presence or absence of
C) catalysts,
wherein the modified diphenylmethane diisocyanates (A) are liquid at 23°C and are prepared by reacting
A1) a diphenylmethane diisocyanate isomer mixture which, based on 100 parts by weight, consists of
A11) 60 - 90 parts by weight of 4,4'-diphenylmethane diisocyanate,
A12) 40 - 8 parts by weight of 2,4'-diphenylmethane diisocyanate and
A13) 0 - 5 parts by weight of 2,2'-diphenylmethane diisocyanate with
A2) at least one polyoxypropylene polyol with a molecular weight of from 350 to 800, obtained using glycerol, trimethylolpropane or a mixture of glycerol and trimethylolpropane as starter molecules,
in the NCO:OH group ratio of 2.5:1 to 15:1.

2. A process for preparing crystal clear, autoclaveable, essentially compact polyurethane embedding compositions by reacting
A) modified diphenylmethane diisocyanates with
B) a mixture of compounds having at least two reactive hydrogen atoms, which consists of
B1) at least one polyhydroxy compound with a molecular weight of from 1000 to 8500 and a functionality of from 2 to 4, selected from the group of polyetherols, polyesterols, hydroxyl-containing polyacetals and hydroxyl-containing polycarbonates,
B2) at least one dihydroxy compound selected from the group of alkanediols having 2 to 10 C atoms, of (alkyl)cycloalkanediols having 6 to 19 C atoms, of glycols which contain bound ester or ether bridges and have molecular weights of up to 378 and of oxyalkylene glycols having 4 to 8 C atoms and
B3) at least one crosslinker having a hydroxyl number of from 230 to 1900 from the group of low molecular weight 3- to 8-hydric alcohols, trialkanolamines, polyoxyalkylene polyols which have been started with alkylenediamines and have hydroxyl numbers of from 350 to 950, or polyoxyalkylene polyols which have been started with low molecular weight 3- to 8-hydric alcohols and have hydroxyl numbers of from 944 to 1500,
in the equivalence ratio of NCO groups of (A) to the total of the reactive hydrogen atoms of (B) of 1:0.9 - 1.3
in the presence or absence of
C) catalysts,
wherein the modified diphenylmethane diisocyanates (A) are liquid at 23°C and are prepared by reacting
A1) a diphenylmethane diisocyanate isomer mixture which, based on 100 parts by weight, consists of
A11) 60 - 90 parts by weight of 4,4'-diphenylmethane diisocyanate,
A12) 40 - 8 parts by weight of 2,4'-diphenylmethane diisocyanate and
A13) 0 - 5 parts by weight of 2,2'-diphenylmethane diisocyanate with
A2) at least one polyoxypropylene polyol with a molecular weight of from 350 to 800, obtained using glycerol, trimethylolpropane or a mixture of glycerol and trimethylolpropane as starter molecules,
in the NCO:OH group ratio of 2.5:1 to 15:1.

3. The use of a crystal clear, autoclaveable, essentially compact polyurethane embedding composition as claimed in claim 1 for embedding hollow fibers composed of, preferably, polysulfones, polycarbonates or cellulose in dialyzers, for producing medical technology articles and for bonding bioceramic coatings to endoprostheses.

## Revendications

1. Masses à couler de polyuréthane, essentiellement compactes, claires et stérilisables à la vapeur chaude, produites par réaction entre
A) des diphénylméthanediisocyanates modifiés et
B) un mélange de composés comportant au moins deux atomes d'hydrogène réactifs, constitué par
B1) au moins un composé polyhydroxyle présentant un poids moléculaire compris entre 1000 et 8500 et une fonctionnalité de 2 à 4, choisi dans le groupe des polyétherols, des polyesterols, des polyacétals contenant des groupes hydroxyle et des polycarbonates contenant des groupes hydroxyle,
B2) au moins un composé dihydroxy, choisi dans le groupe des alcanediols comportant 2 à 10 atomes de carbone, des (alkyl)cycloalcanediols comportant 6 à 19 atomes de carbone, des glycols contenant des ponts ester et éther fixés, présentant des poids moléculaires allant jusqu'à 378 et des oxyalkylèneglycols comportant 4 à 8 atomes de carbone et
B3) au moins un agent de réticulation contenant des groupes hydroxyle présentant un indice d'hydroxyle compris entre 230 et 1900 et une fonctionnalité de 3 à 8, le rapport molaire B1 : B2 : B3 étant compris entre 1:0,01 à 48:0,01 à 32,
dans un rapport d'équivalents entre les groupes NCO de (A) et la somme des atomes d'hydrogène réactifs de (B) entre 1:0,09 à 1,3,
en présence ou en l'absence de
C) catalyseurs,
caractérisées en ce que les diphénylméthanediisocyanates modifiés (A) sont liquides à 23 °C et sont produits par réaction entre
A1) un mélange d'isomères de diphénylméthane-diisocyanate, qui est constitué, par rapport à 100 parties en poids,
A11) par 60 à 90 parties en poids de 4,4'-diphénylméthanediisocyanate,
A12) par 40 à 8 parties en poids de 2,4'-diphénylméthanediisocyanate et
A13) par 0 à 5 parties en poids de 2,2'-diphénylméthanediisocyanate et
A2) au moins un polyoxypropylènepolyol présentant un poids moléculaire compris entre 350 et 800, obtenu en utilisant de la glycérine, du triméthylolpropane ou un mélange de glycérine et de triméthylolpropane comme molécule d'amorçage,
dans un rapport entre les groupes NCO et OH compris entre 2,5:1 à 15:1.

2. Procédé pour la production de masses à couler de polyuréthane, essentiellement compactes, claires et stérilisables à la vapeur chaude par réaction entre
A) des diphénylméthanediisocyanates modifiés et
B) un mélange de composés comportant au moins deux atomes d'hydrogène réactifs, constitué par
B1) au moins un composé polyhydroxyle présentant un poids moléculaire compris entre 1000 et 8500 et une fonctionnalité de 2 à 4, choisi dans le groupe des polyétherols, des polyesterols, des polyacétals contenant des groupes hydroxyle et des polycarbonates contenant des groupes hydroxyle,
B2) au moins un composé dihydroxy, choisi dans le groupe des alcanediols comportant 2 à 10 atomes de carbone, des (alkyl)cycloalcanediols comportant 6 à 19 atomes de carbone, des glycols contenant des ponts ester et éther fixés, présentant des poids moléculaires allant jusqu'à 378 et des oxyalkylèneglycols comportant 4 à 8 atomes de carbone et
B3) au moins un agent de réticulation présentant un indice d'hydroxyle compris entre 230 et 1900 du groupe des alcools trivalents à octavalents à bas poids moléculaire, des trialkanolamines, des polyoxyalkylènepolyols amorcés par des alkylènediamines présentant des indices d'hydroxyle compris entre 350 et 950 ou des polyoxyalkylènepolyols amorcés par des alcools trivalents à octavalents à bas poids moléculaire présentant des indices d'hydroxyle de 944 à 1500
B3) au moins un agent de réticulation contenant des groupes hydroxyle présentant un indice d'hydroxyle de 230 à 1900 et une fonctionnalité de 3 à 8, le rapport molaire B1 : B2 : B3 étant compris entre 1:0,01 à 48:0,01 à 32,
dans un rapport d'équivalents entre les groupes NCO de (A) et la somme des atomes d'hydrogène réactifs de (B) entre 1:0,9 à 1,3,
en présence ou en l'absence de
C) catalyseurs,
caractérisées en ce que les diphénylméthanediisocyanates modifiés (A) sont liquides à 23 °C et sont produits par réaction entre
A1) un mélange d'isomères de diphénylméthane-diisocyanate, qui est constitué, par rapport à 100 parties en poids,
A11) par 60 à 90 parties en poids de 4,4'-diphénylméthanediisocyanate,
A12) par 40 à 8 parties en poids de 2,4'-diphénylméthanediisocyanate et
A13) par 0 à 5 parties en poids de 2,2'diphénylméthanediisocyanate et
A2) au moins un polyoxypropylènepolyol présentant un poids moléculaire compris entre 350 et 800, obtenu en utilisant de la glycérine, du triméthylolpropane ou un mélange de glycérine et de triméthylolpropane comme molécule d'amorçage,
dans un rapport entre les groupes NCO et OH compris entre 2,5:1 à 15:1.

3. Utilisation des masses à couler de polyuréthane, essentiellement compactes, claires et stérilisables à la vapeur chaude selon la revendication 1 pour l'incorporation de fibres creuses, de préférence de polysulfones, de polycarbonates ou de cellulose, dans des appareils de dialyse pour la fabrication d'articles médico-techniques ainsi que pour la fixation de revêtements en biocéramique sur des endoprothèses.
